(19) Europäisches Patentamt
European Patent Office
Office européen des brevets

(11) Publication number: **0 034 471**
**A1**

(12) # EUROPEAN PATENT APPLICATION

(21) Application number: 81300579.0

(22) Date of filing: 12.02.81

(51) Int. Cl.³: C 07 D 401/04
C 07 D 401/14

(30) Priority: 19.02.80 FI 800481

(43) Date of publication of application: 26.08.81 Bulletin 81/34

(84) Designated Contracting States: AT BE CH DE FR GB IT LI LU NL SE

(71) Applicant: Orion-yhtymä Oy
PL 19
00101 Helsinki 10(FI)

(72) Inventor: Honkanen, Erkki Juhani
Vanha Viertotie 1 A 13
SF-00300 Helsinki 30(FI)

(72) Inventor: Hietava, Maija Marjaana
Kivihaantie 5 A 11
SF-00310 Helsinki 31(FI)

(72) Inventor: Kairisalo, Pekka Juhani
Uiskotie 25
SF-00950 Helsinki 95(FI)

(72) Inventor: Nore, Pentti Tapio
Marunakuja 4 B 45
SF-00840 Helsinki 84(FI)

(72) Inventor: Karppanen, Heikki Olavi
Lehdesniityntie 3 I 145
SF-00340 Helsinki 34(FI)

(72) Inventor: Paakkari, Asko Tuomo Iiari
Soukanahde 7 F 115
SF-02360 Espoo 36(FI)

(74) Representative: Warden, John C. et al,
R.G.C. Jenkins & Co. Chancery House 53/64 Chancery Lane
London WC2A 1QU(GB)

(54) **Novel piperidinoquinazolines and a process for the preparation thereof.**

(57) Novel substituted 2-piperidino-4-amino-6,7-dimethoxy-quinazolines having the formula

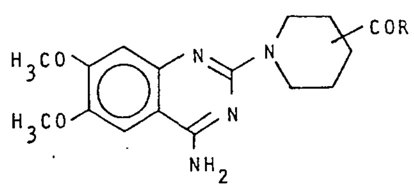

wherein R is an alkyl containing 1 to 5 carbons, a cycloalkyl containing 3 to 7 carbons, benzyl, an alkoxy containing 1 to 5 carbons, benzyloxy, or a group $-NR^1R^2$, wherein $R^1$ or $R^2$ is hydrogen or a chained alkyl containing 4 to 5 carbons or wherein $R^1$ and $R^2$, together with the nitrogen atom, form a cyclic amino group containing 3 to 7 carbons, which amino group may be substituted by one or two lower-alkyl groups, or their pharmaceutically acceptable salts.

EP 0 034 471 A1

Novel Piperidinoquinazolines and a Process for the Preparation thereof

The present invention relates to novel substituted 2-piperidino-4-amino-6,7-dimethoxyquinazolines. The compounds have the general formula

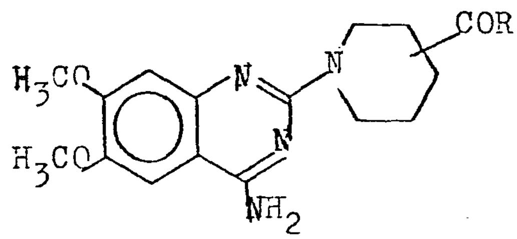

I

wherein R is an alkyl containing 1 to 5 carbons, a cycloalkyl containing 3 to 7 carbons, benzyl, an alkoxy containing 1 to 5 carbons, benzyloxy, or a group $-NR^1R^2$, wherein $R^1$ or $R^2$ is hydrogen or a chained alkyl containing 4 to 5 carbons or wherein $R^1$ and $R^2$, together with a nitrogen atom, form a cyclic amino group containing 3 to 7 carbons, which group may be substituted by one or two lower-alkyl groups.

These compounds have a lowering effect on the blood pressure.

It is known that certain esters and amides of piperidine-carboxylic acid lower the blood pressure (A.P. Swain & S.K. Naegele, J.Am.Chem.Soc. 79 (1957) 5250; J. Biel et al., ibid 79 (1957) 6181; J. Sam et al., ibid 81 (1959) 710). On the other hand, it is known that 4-amino-6,7-dimethoxyquinazoline also lowers the blood pressure (H.-J. Hess, Clinical Symp. Proceed., San Francisco, May 16-17, 1974). 4-amino-6,7-dimethozyquinazoline appears also as a structural component in 2-(4-(2-furoyl)-piperazin-1-yl)-4-amino-6,7-dimethoxyquinazoline or prazosin, which is a well-known antihypertensive agent.

2-(4-benzoyl)piperidino- 4-amino-6,7-dimethoxyquinazoline and 2-(4-(2-thienoyl)piperidino)-4-amino-6,7-dimethoxyquinazoline, for example, are known in the French patent specifications 2 350 101 and 2 389 621, and further, 2-(2-diethylamino-carbonylpiperidino)-4-amino-6,7-dimethoxyquinazoline, 2-(4-n-butyyliaminocarbonylpiperidino)-4-amino-6,7-dimethoxyquinazoline and 2-(4-morpholinocarbonyl-piperidino)-4-amino-6,7-dimethoxyquinazoline are known in the British patent application 2 021 108.

The major side effect of prazosin is the first dose phenomenon which consists in an acute reaction characterized by dizziness, palpitations and sometimes loss of consciousness for periods of time which may range from a few minutes to over an hour (I. Cavero and A.G. Roach: The pharmacology of prazosin, a novel antihypertensive agent. Life Sciences 27, 1525-1540, 1980). The strong initial fall of blood pressure is also observed in animal experiments.

The great advantage of the new compounds according to the present invention over prazosin is the lack of a strong initial fall of blood pressure, but a gradual and long-lasting lowering of the blood pressure level. Therefore no first dose phenomenon can be expected by the use of these drugs.

The compounds of the formula I are prepared so that either

a) a 2-substituted 4,5-dimethozyaniline of the formula II

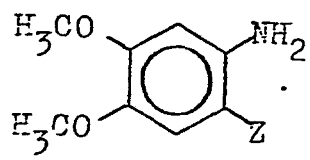

II

wherein Z is a cyano or amidino group, is reacted with an acylpiperidine derivative of the formula III

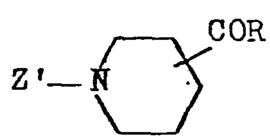

III

wherein Z' is a cyano or amidino group,

b) a 4-amino-6,7-dimethoxy-2-quinazolinyl-halogenide of the formula IV

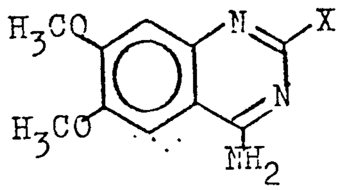

IV

wherein X is chlorine or bromine and whose amino group is protected if desired, is reacted with an acylpiperidine of the formula V

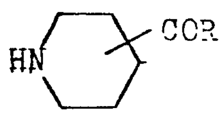

V

and the protective group, if any, is removed,

c) a S-alkyl-N-cyano-N'-(3,4-dimethoxyphenyl)-isothiourea of the formula VI

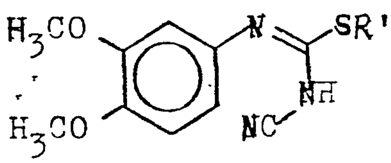

VI

wherein R' is a lower-alkyl group, is reacted with an acylpiperidine of the formula V,

d) an alkyl N-(2-cyano-4,5-dimethoxyphenyl)-acyl-piperidinothioformamidate derivative of the formula VII

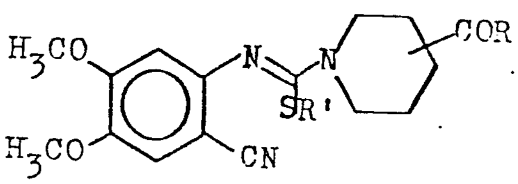

VII

wherein R' is a lower-alkyl group, is reacted with ammonia, ammonium halogenide, urea, or urea hydrohalogenide, or

e) for the preparation of such compounds of the formula I in which R is the group $-NR^1R^2$, a 2-(acylpiperidino)-4-amino-6,7-dimethoxyquinazoline of the formula VIII

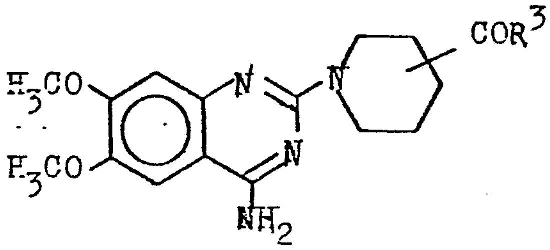

VIII

wherein $COR^3$ is an activated carboxyl group such as ester, mixed anhydride or acid halogenide, is reacted with an amine of the formula IX.

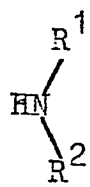

IX

If desired, the final product may be converted to a pharmaceutically acceptable salt, such as hydrochloride.

The invention is also concerned with optically active 2- or 3-acylpiperidines. The resolution may be performed either on the starting materials of the formula V, on the intermediates prepared from them, or on the final products of the formula I.

The reaction in accordance with the process alternative a) is preferably performed at an elevated temperature, in a polar aprotic solvent, such as dimethylformamide, dimethylacetamide or dimethylsulphoxide, and in the presence of an basic catalyst, such as alkalimetalalkoxide, -amide, -hydride or -alkyl. Among the compounds of the formula II, 2-amino-4,5-benzonitrile is a known compound. The corresponding 2-amidino compound is obtained from it by treatment with hydroxylamine followed by catalytic hydrogenation. Compounds of the formula III wherein Z' is a cyano group are obtained from corresponding acylpiperidines with the aid of cyanogenbromide. Compounds in which Z' is an amidino group are obtained from corresponding methyl acylpiperidinothioformamidates with the aid of ammonia.

The reaction in accordance with the process alternative b) is preferably performed at an elevated temperature and in an inert solvent, such as isoamylalcohol. Halogenides of the formula IV to be used as the starting material can be prepared according to the process described in the U.S. Patent 3.511 836. The amino group can be protected by means of conventional methods, e.g. in the way suggested in the Finnish Patent Application 771 741.

Piperidines of the formula V wherein R is ethoxy or amino are readily available compounds. Compounds where R is another alkoxy, benzyloxy or group $-NR^1R^2$ are obtained from the ethyl ester by conventional methods.

Piperidines of the formula V wherein R is alkyl, cycloalkyl or benzyl are obtained by reacting N-benzyl-piperidinecarbonitrile with alkyl-, cycloalkyl- or benzyl lithium or -magnesium halogenide and removing the N-benzyl group by catalytic hydrogenation. N-benzyl-piperidinecarbonitrile is obtained by reacting carbamoylpiperidine with benzyl chloride and then with acetanhydride, thionyl chloride or diphosphorous pentoxide.

The reaction in accordance with the process alternative c) is preferably performed at an elevated temperature and in an inert solvent, such as diglycoldimethylether. The starting materials of formula VI can be prepared by reacting 3,4-dimethoxyaniline with dimethylester of cyanamid-dithiocarboxylic acid.

The reaction in accordance with the process alternative d) is preferably performed at an elevated temperature and in polar solvent, such as formamide. The compounds of formula VII are novel, and they can be prepared advantageously by means of the process described in the Finnish Patent Application No. 800 482. Therein 2-amino-4,5-dimethoxybenzonitrile is reacted with thiophosgene to form 2-isothiocyanato-4,5-dimethoxybenzonitrile, which is reacted with acyl piperidine according to the formula V followed by alkylation with alkyl iodide to form the compound according to the formula VII.

The reaction in accordance with the process alternative e) is performed at an elevated temperature and in an inert solvent or, if possible, by using the reacting compound of the formula IX as a solvent. The compounds of the formula VIII are novel, and they can be prepared advantageously by means of the process in accordance with the process alternative d).

Especially the method according to alternative d) has been found to be profitable. It is also suitable for the production of the compounds of the formula I in the industrial scale. All steps of this synthesis starting from 2-amino-4,5-dimethoxybenzonitrile can be carried out in the same reactor without isolation of the intermediates. When ammonium chloride or urea hydrochloride is used as the reagent in the last step the product is obtained directly as the hydrochloride which is the prefered pharmaceutical form in most cases. More impurities are formed if the hydrochloride salt is precipitated with hydrochloric acid from the solution of the corresponding free base. The removal of these impurities may be quite troublesome.

The effect of the compounds when administered intravenously was studied with male Wistar rats, 240-290 g,which were anaesthetised with urethane (1.5 g/kg intraperitoneally). A heating pad which is controlled by a thermostate connected to the colonic thermistor probe was placed underneath the rats to keep the body temperature at 38$^{o}$C. The trachea was cannulated with a polyethylene tube and the rats were allowed to breathe spontaneously. A femoral vein was cannulated for intravenous injections of drugs. The blood pressure was measured from the femoral artery connected to a pressure transducer (MP-15, Micron Instruments, Inc.). The pressure signals were amplified with a pressure conditioner (HP 8805D). The mean arterial pressure was recorded on paper with a chart recorder.

The effect of the drugs by oral administration on the spontaneous hypertension in conscious rats was studied by using a blood pressure recorder (model no. 8002e, W+W Electronics Inc. Basel). Spontaneously hypertensive rats of the

Okamoto-Aoki strain were used. The blood pressure was measured indirectly by a tail cuff method. After keeping the rats for 30 min. at 36°C in a warming chamber to permit pulse waves be recorded, the rats were placed in a plexyglass cylinder, and six consecuetive systolic arterial pressure measurements were obtained from each rat. The arithmetic mean of these readings was taken as the systolic blood pressure.

The effect of the compounds according to the present invention was compared with that of prazosin and two compounds disclosed in the British published patent application 2 021 108, viz. 2-(4-diethylaminocarbonylpiperidino)-4-amino-6,7-dimethoxyquinazoline (reference compound A) and 2-(4-morpholinocarbonylpiperidino)-4-amino-6,7-dimethoxyquinazoline (reference compound B).

The lowering effect on blood pressure of the compounds according to the invention both in intravenous (i.v.) and oral (p.o.) administration is presented in the enclosed table. A dose of 0.1 µmol/kg was injected intravenously and the maximal decrease of the blood pressure (mmHg) was measured. A dose of 0.5 µmol/rat was administered orally and the decrease of the blood pressure was measured 1, 2 and 5 hours after the administration.

Table: The lowering effect on the blood pressure of the compounds according to formula X by rats.

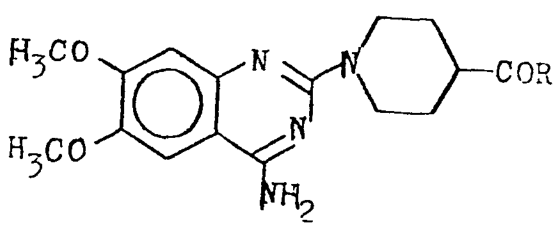

X

| R | The decrease of the blood pressure (mmHg) induced by the dose of 0.1 μmol/kg i.v. | The decrease of the blood pressure (mmHg) induced by the dose of 0.5 μmol/rat p.o. | | |
|---|---|---|---|---|
| | | 1h | 2h | 5h |
| neopentyloxy | 44 | 22 | 21 | 21 |
| amino | 27 | 20 | 22 | 20 |
| t-butylamino | 28 | 15 | 25 | 28 |
| piperidino | 32 | 25 | 22 | 17 |
| 1-azacycloheptyl | 47 | 23 | 27 | 25 |
| 1-azacyclooctyl | 23 | 18 | 23 | 29 |
| 2-methylpiperidino | 31 | 15 | 25 | 28 |
| Reference compounds: | | | | |
| prazosin | 36 | 26 | 31 | 24 |
| A(R = diethylamino) | 37 | 24 | 16 | 10 |
| B(R = morpholino) | 30 | 7 | 5 | -2 |

The following examples illustrate the invention.

EXAMPLE 1. 2-(3-pyrrolidinocarbonylpiperidino)-4-amino-6,7-dimethoxyquinazoline

To a solution of 1.78 g (0.01 mole) of 2-amino-4,5-dimethoxybenzonitrile and 2.07 g (0.01 mole) of 1-cyano-3-pyrrolidinocarbonylpiperidine in 25 ml of dry dimethylacetamide was added 0.5 g (0.02 mole) of sodium hydride. The solution was heated in a nitrogen atmosphere for 5 hours at 80°C. After cooling, 50 ml of water was added, and the product was extracted with chloroform. The extract was washed several times with water, the solvent removed under reduced pressure, and the residue crystallized from a mixture of ethanol-water (80:20). 1.2 g of 2-(3-pyrrolidinocarbonylpiperidino)-4-amino-6,7-dimethoxyquinazoline was obtained; mp. 134-136°C, yield 30 %.

EXAMPLE 2. 2-(4-pyrrolidinocarbonylpiperidino)-4-amino-6,7-dimethoxyquinazoline hydrochloride

A solution of 2.4 g (0.01 mole) of 2-chloro-4-amino-6,7-dimethoxyquinazoline and 1.82 g (0.01 mole) of 4-pyrrolidinocarbonylpiperidine in 30 ml of isoamylalcohol was boiled under reflux for 18 hours. After cooling, the precipitated product was filtered, washed with ether and dried. 1.0 g of 2-(4-pyrrolidinocarbonylpiperidino)-4-amino-6,7-dimethoxyquinazoline hydrochlororide was obtained; mp. 201-203°C, yield 24 %.

EXAMPLE 3. 2-(4-pyrrolidinocarbonylpiperidino)-4-amino-6,7-dimethoxyquinazoline hydrochloride

A solution of 1.0 g (0.004 mole) of S-methyl-N-cyano-N'-(3,4-dimethoxyphenyl)isothiourea and 0.78 g (0.004 mole) of 4-pyrrolidinocarbonylpiperidine in 15 ml of diglycoldimethylether was boiled under reflux for 5 hours. After cooling water was added, and the solution extracted with chloroform. The extract was washed with water and dried. An ether solution of hydrogen chloride was added to the extract, whereby the product was crystallized. On recrystallization from a mixture of water-ethanol (80:20), 0.35 g of 2-(4-pyrrolidinocarbonylpiperidino)-4-amino-6,7-dimethoxyquinazoline hydrochloride was obtained; mp. 201-203°C, yield 21 %.

EXAMPLE 4. 2-(4-ethoxycarbonylpiperidino)-4-amino-6,7-dimethoxyquinazoline hydrochloride

A mixture of 39.1 g (0.1 mole) of methyl N-(2-cyano-4,5-dimethoxyphenyl) (4-ethoxycarbonylpiperidino)thioformamidate and 160 g (3 moles) of ammonium chloride (as an alternative: 3 moles of urea hydrochloride) in 400 ml of formamide was heated for 24 hours in a nitrogen stream while stirring at 120°C, cooled, and 400 ml of water was added. The precipitated product was filtered, washed with cold water and acetone, and dried. 20.0 g of 2-(4-ethoxycarbonylpiperidino)-4-amino-6,7-dimethoxyquinazoline hydrochloride was obtained; mp. 260-265°C, yield 50 %.

EXAMPLE 5. 2-(4-pyrrolidinocarbonylpiperidino)-4-amino-6,7-dimethoxyquinazoline

A solution of 3.6 g (0.01 mole) of 2-(4-ethoxycarbonyl)-piperidino-4-amino-6,7-dimethoxyquinazoline in 10 ml of pyrrolidine was boiled for 42 hours under reflux. The solvent was removed under reduced pressure, and the residue crystallized from a mixture of ethanol - water (80:20). 3.6 g of 2-(4-pyrrolidinocarbonylpiperidino)-4-amino-6,7-dimethoxyquinazoline was obtained; mp. 312-314°C, yield 94 %.

EXAMPLE 6. 2-(3-pyrrolidinocarbonylpiperidino)-4-amino-6,7-dimethoxyquinazoline

A mixture of 41.6 g (0.1 mole) of methyl N-(2-cyano-4,5-dimethoxyphenyl)-(3-pyrrolidinocarbonylpiperidino)thioformamidate and 160 g of ammonium chloride in 400 ml of formamide was heated for 24 hours in a nitrogen stream at 120°C, 400 ml of water was added, the mixture was made basic with concentrated ammonia and extracted with chloroform. The extract was washed with water and the solvent removed under reduced pressure. The residue was crystallized from a mixture of ethanol - water (80:20). 30.8 g of 2-(3-pyrrolidinocarbonylpiperidino)-4-amino-6,7-dimethoxyquinazoline was obtained; mp. 134-136°C, yield 80 %.

EXAMPLE 7. 2-(4-pyrrolidinocarbonylpiperidino)-4-amino-6,7-dimethoxyquinazoline hydrochloride

When using 41.6 g of methyl N-(2-cyano-4,5-dimethoxyphenyl)-(4-pyrrolidinocarbonylpiperidino)-thioformamidate as the starting material and proceeding in the same way as described in example 4, 35.8 g of 2-(4-pyrrolidinocarbonylpiperidino)-4-amino-6,7-dimethoxyquinazoline hydrochloride was obtained; mp. 201-203°C, yield 85 %.

EXAMPLE 8. 2-(4-neopentyloxycarbonylpiperidino)-4-amino-6,7-dimethoxyquinazoline hydrochloride

When using 43.3 g of methyl N-(2-cyano-4,5-dimethoxyphenyl)-(4-neopentyloxycarbonylpiperidino)thioformamidate as the starting material and proceeding in the same way as described in example 4, 16.0 g of 2-(4-neopentyloxycarbonylpiperidino)-4-amino-6,7-dimethoxyquinazoline hydrochloride was obtained; mp. 269-272°C, yield 37 %.

EXAMPLE 9. 2-(4-piperidinocarbonylpiperidino)-4-amino-6,7-dimethoxyquinazoline

When using 43 g of methyl N-(2-cyano-4,5-dimethoxyphenyl)-(4-piperidinocarbonylpiperidino)thioformamidate as the starting material and proceeding in the same way as described in example 6, 24 g of 2-(4-piperidinocarbonylpiperidino)-4-amino-6,7-dimethoxyquinazoline was obtained; mp. 125-128$^{o}$C, yield 61 %.

EXAMPLE 10. (+)-2-(3-ethoxycarbonylpiperidino)-4-amino-6,7-dimethoxyquinazoline

When using 39.1 g of (+)-methyl N-(2-cyano-4,5-dimethoxyphenyl)-(3-ethoxycarbonylpiperidino)thioformamidate as the starting material and proceeding in the same way as described in example 6, 24.0 g of (+)-2-(3-ethoxycarbonylpiperidino)-4-amino-6,7-dimethoxyquinazoline was obtained, mp. 227-230$^{o}$C, $[\alpha]_D^{20}$ +94.2$^{o}$, yield 67 %.

EXAMPLE 11. (-)-2-(3-ethoxycarbonylpiperidino)-4-amino-6,7-dimethoxyquinazoline

When using 39.1 g of (-)-methyl N-(2-cyano-4,5-dimethoxyphenyl)-(3-ethoxycarbonylpiperidino)thioformamidate as the starting material and proceeding in the same way as described in example 6, 25.0 g of (-)-2-(3-ethoxycarbonylpiperidino)-4-amino-6,7-dimethoxyquinazoline was obtained, mp. 227-229$^{o}$C, $[\alpha]_D^{20}$ -96.0$^{o}$ (0.2 N HCl, c = 5 g/100 ml), yield 69 %.

EXAMPLE 12. 2-(4-(1-azetidinyl)carbonylpiperidino)-4-amino-6,7-dimethoxyquinazoline

When using 2-(4-ethoxycarbonyl)-piperidino-4-amino-6,7-dimethoxyquinazoline and azetidine as the starting material and proceeding in the same way as described in example 5, 2-(4-(1-azetidinyl)carbonylpiperidino)-4-amino-6,7-dimethoxyquinazoline was obtained; mp. 238-244$^{o}$C, yield 80 %.

EXAMPLE 13. 2-(4-carbamoylpiperidino)-4-amino-6,7-dimethoxyquinazoline hydrochloride

A mixture of 180 g (0.5 mole) of methyl N-(2-cyano-4,5-dimethoxyphenyl) (4-carbamoylpiperidino)thioformamidate and 780 g ammoniumchloride in 2600 ml of formamide was heated

while stirring for 24 hours in a nitrogen flow at 120°C. After cooling 1300 ml of water was added, and the precipitated product was filtered, washed with water and acetone, and dried. 130 g of 2-(4-carbamoylpiperidino)-4-amino-6,7-dimethoxyquinazoline hydrochloride was obtained; mp. 273-276°C, yield 80 %.

EXAMPLE 14. 2-(4-tert-butylcarbamoylpiperidino)-4-amino-6,7-dimethoxyquinazoline hydrochloride

A mixture of 1.3 g (0.003 mole) of methyl N-(2-cyano-4,5-dimethoxyphenyl) (4-tert-butylcarbamoylpiperidino)thioformamidate and 39 g of ammonium chloride in 10 ml of formamide was heated while stirring for 24 hours in nitrogen flow. After cooling water and concentrated ammonia were added until the pH was 9.0. The solution was extracted with chloroform. The extract was washed with water and evaporated under reduced pressure. The residue was dissolved in ethanol and gaseous hydrogen chloride was added to precipitate the hydrochloride salt. 0.4 g of 2-(4-tert-butylcarbamoylpiperidino)-4-amino-6,7-dimethoxyquinazoline hydrochloride was obtained; mp. 129-132°C, yield 31 %.

EXAMPLE 15. 2-(4-(2-methylpiperidino)carbonylpiperidino)-4-amino-6,7-dimethoxyquinazoline hydrochloride

A mixture of 44.5 g (0.1 mole) of methyl N-(2-cyano-4,5-dimethoxyphenyl) (4-(2-methylpiperidino)carbonylpiperidino)-thioformamidate and 160 g of ammonium chloride in 500 ml of formamide was heated for 24 hours at 120°C. The product was isolated as described in example 6. 29.4 g of 2-(4-(2-methylpiperidino)carbonylpiperidino)-4-amino-6,7-dimethoxyquinazoline hydrochloride was obtained; mp 140-143°C, yield 71 %.

EXAMPLE 16. 2-(4-(azacyclohept-1-yl-carbonyl)piperidino)-4-amino-6,7-dimethoxyquinazoline hydrochloride

A mixture of 89 g (0.2 mole) of methyl N-(2-cyano-4,5-dimethoxyphenyl) (4-(azacyclohept-1-yl-carbonyl)piperidino)-thioformamidate and 300 g of ammonium chloride in 1000 ml of formamide was heated for 24 hours at 120°C. The product was isolated as in example 6. 45.5 g of 2-(4-(azacyclohept-1-yl-carbonyl)piperidino)-4-amino-6,7-dimethoxyquinazoline hydrochloride was obtained; mp. 234-235°C, yield 55 %.

EXAMPLE 17. 2-(4-(azacyclooct-1-yl-carbonyl)piperidino-4-amino-6,7-dimethoxyquinazoline hydrochloride

When using 22.95 g (0.05 mole) of methyl N-(2-cyano-4,5-dimethoxyphenyl) (4-(azacyclooct-1-yl-carbonyl)piperidino)-thioformamidate as the starting material and proceeding in the same way as described in example 6, 12.8 g 2-(4-(azacyclooct-1-yl-carbonyl)piperidino)-4-amino-6,7-dimethoxyquinazoline hydrochloride was obtained; mp. 237-239°C, yield 60 %.

EXAMPLE 18. (+)-2-(3-pyrrolidinocarbonylpiperidino)-4-amino-6,7-dimethoxyquinazoline hydrochloride

When using 2.2 g (0.0053 mole) of (+)-methyl N-(2-cyano-4,5-dimethoxyphenyl)-(3-pyrrolidinocarbonylpiperidino)thioformamidate and proceeding as in example 6, 1.8 g of (+)-2-(3-pyrrolidinocarbonylpiperidino)-4-amino-6,7-dimethoxyquinazoline hydrochloride was obtained; mp. 184-188°C, $\alpha_D^{20}$ +3 ±0.5°, yield 81 %.

EXAMPLE 19. (-)-2-(3-pyrrolidinocarbonylpiperidino)-4-amino-6,7-dimethoxyquinazoline hydrochloride

When using 2.5 g (0.006 mole) of (-)-methyl N-(2-cyano-4,5-dimethoxyphenyl)-(3-pyrrolidinocarbonylpiperidino)thioformamidate as the starting material and proceeding as in example 6, 2.1 g of (-)-2-(3-pyrrolidinocarbonylpiperidino)-4-amino-6,7-dimethoxyquinazoline hydrochloride was obtained; mp. 182-188°C, $\alpha_D^{20}$ -4 ±0.5°, yield 83 %.

EXAMPLE 20. 2-(4-(2,5-dimethylpyrrolidinocarbonyl)-piperidino)-4-amino-6,7-dimethoxyquinazoline hydrochloride

When using 3.8 g (0.006 mole) of methyl N-(2-cyano-4,5-dimethoxyphenyl) (4-(2,5-dimethylpyrrolidinocarbonyl)-piperidino)thioformamidate as the starting material and proceeding as in example 6, 0.50 g of 2-(4-(2,5-dimethylpyrrolidinocarbonyl)piperidino)-4-amino-6,7-dimethoxyquinazoline hydrochloride was obtained; mp. 248-252°C, yield 19 %.

EXAMPLE 21. 2-(4-(2,6-dimethylpiperidinocarbonyl)-piperidino)-4-amino-6,7-dimethoxyquinazoline hydrochloride

When using 3.7 g (0.008 mole) of methyl N-(2-cyano-4,5-dimethoxyphenyl) (4-(2,6-dimethylpiperidinocarbonyl)-piperidino)thioformamidate and proceeding as in example 6, 0.6 g of 2-(4-(2,6-dimethylpiperidinocarbonyl)piperidino)-4-amino-6,7-dimethoxyquinazoline hydrochloride; mp. 270-274°C, yield 16 %.

EXAMPLE 22. 2-(4-acetylpiperidino)-4-amino-6,7-dimethoxyquinazoline hydrochloride

When using 10.0 g (0.028 mole) of methyl N-(2-cyano-4,5-dimethoxyphenyl) (4-acetylpiperidino)thioformamidate as starting material and proceeding as in example 6, 2.3 g of 2-(4-acetylpiperidino)-4-amino-6,7-dimethoxyquinazoline hydrochloride was obtained; mp. 140-142°C, yield 22 %.

EXAMPLE 23. 2-(4-propionylpiperidino)-4-amino-6,7-dimethoxyquinazoline hydrochloride

When using 5.6 g (0.015 mole) of methyl N-(2-cyano-4,5-dimethoxyphenyl) (4-propionylpiperidino)thioformamidate as starting material and proceeding as in example 6, 1.9 g of 2-(4-propionylpiperidino)-4-amino-6,7-dimethoxyquinazoline hydrochloride was obtained; mp. 167-170°C, yield 32 %.

EXAMPLE 24. 2-(4-valeroylpiperidino)-4-amino-6,7-dimethoxyquinazoline hydrochloride

When using 9.2 g (0.023 mole) of methyl N-(2-cyano-4,5-dimethoxyphenyl) (4-n-valeroylpiperidino)thioformamidate as starting material and proceeding as in example 6, 4.7 g of 2-(4-n-valeroylpiperidino)-4-amino-6,7-dimethoxyquinazoline hydrochloride was obtained; mp. 196-199°C, yield 50 %.

EXAMPLE 25. 2-(4-cyclohexylcarbonylpiperidino)-4-amino-6,7-dimethoxyquinazoline hydrochloride

When using 9.50 g (0.022 mole) of methyl N-(2-cyano-4,5-dimethoxyphenyl) (4-cyclohexylcarbonylpiperidino)-thioformamidate as starting material and proceeding as in example 6, 3.7 g of 2-(4-cyclohexylcarbonylpiperidino)-4-amino-6,7-dimethoxyquinazoline hydrochloride was obtained; mp. 290-293°C, yield 38 %.

What is claimed is

1. Novel substituted 2-piperidino-4-amino-6,7-dimethoxyquinazolines having the formula I

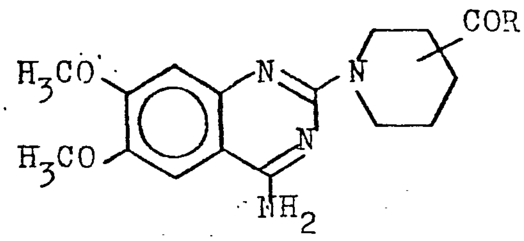

I

wherein R is an alkyl containing 1 to 5 carbons, a cycloalkyl containing 3 to 7 carbons, benzyl, an alkoxy containing 1 to 5 carbons, benzyloxy, or a group $-NR^1R^2$, wherein $R^1$ or $R^2$ is hydrogen or a chained alkyl containing 4 to 5 carbons or wherein $R^1$ and $R^2$, together with the nitrogen atom, form a cyclic amino group containing 3 to 7 carbons, which amino group may be substituted by one or two lower-alkyl groups, or their pharmaceutically acceptable salts.

2. Compounds according to claim 1, wherein R is a chained alkoxy containing 3 to 5 carbons.

3. Compounds according to claim 1, wherein R is an amino group.

4. Compounds according to claim 1, wherein R is a cyclic amino group which may be substituted by one or two lower-alkyl groups.

5. A process for the preparation of the compounds according claim 1, characterized in that

a) a 2-substituted 4,5-dimethoxyaniline of the formula II

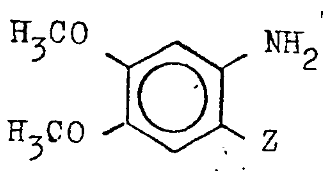

II

wherein Z is a cyano or amidino group, is reacted with an acylpiperidine derivative of the formula III

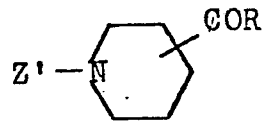

III

wherein Z' is a cyano or amidino group,

b) a 4-amino-6,7-dimethoxy-2-quinazolinyl-halogenide of the formula IV

IV

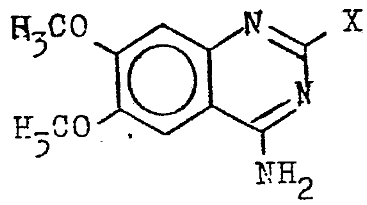

wherein X is chlorine or bromine and whose amino group is protected if desired, is reacted with an acylpiperidine of the formula V

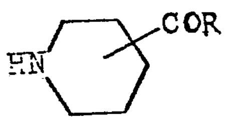

V

and the protective group, if any, is removed,

c) a S-alkyl-N-cyano-N'-(3,4-dimethoxyphenyl)isothiourea of the formula VI

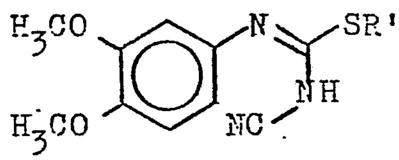

VI

wherein R' is a lower-alkyl group, is reacted with an acylpiperidine of the formula V,

d) an alkyl N-(2-cyano-4,5-dimethoxyphenyl)acylpiperidinothioformamidate derivative of the formula VII

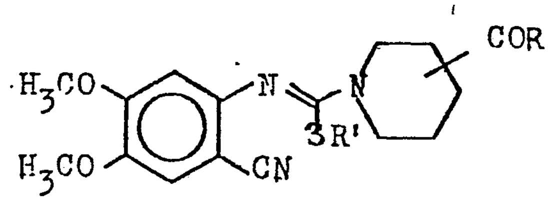

VII

wherein R' is a lower-alkyl group, is reacted with ammonia, ammonium halogenide, urea, or urea hydrohalogenide, or

e) for the preparation of such compounds of the formula I in which R is a group $-NR^1R^2$, a 2-(acylpiperidino)-4-amino-6,7-dimethoxyquinazoline of the formula VIII

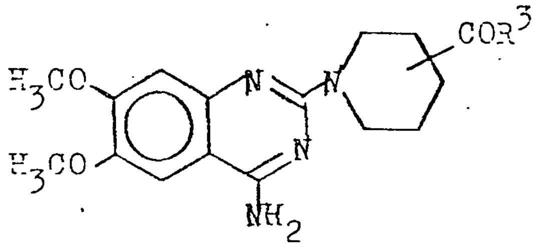

VIII

wherein $COR^3$ is an activated carboxyl group such as ester, mixed anhydride or acid halogenide, is reacted with an amine of the formula IX,

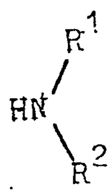

IX

and, if desired, the final product of formula I is converted into its pharmaceutically acceptable salt or, if the final product is a racemic mixture, the optically active isomers are isolated or, if the final product is in the form of a salt, the free base is isolated.

0034471

Application number

European Patent Office

# EUROPEAN SEARCH REPORT

EP 81 30 0579

## DOCUMENTS CONSIDERED TO BE RELEVANT

| Category | Citation of document with indication, where appropriate, of relevant passages | Relevant to claim |
|---|---|---|
| DX | GB - A - 2 021 108 (PFIZER)<br>* Pages 1-6,9,10,14,15 *<br>-- | 1,3-5 |
| D | FR - A - 2 350 101 (SYNTHELABO)<br>* Pages 1,2,3,4 example 11,5-8 *<br>-- | 1,5 |
| | FR - A - 2 321 890 (SYNTHELABO)<br>* Pages 1,2,9,11-16 *<br>---- | 1,2,5 |

CLASSIFICATION OF THE APPLICATION (Int. Cl.³)

C 07 D 401/04
401/14

TECHNICAL FIELDS SEARCHED (Int. Cl.³)

C 07 D 401/04
401/14

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 08-05-1981 | FRANCOIS |

EPO Form 1503.1 06.78